## Europäisches Patentamt
## European Patent Office
## Office européen des brevets

(11) Veröffentlichungsnummer: **0 155 501**
A2

# EUROPÄISCHE PATENTANMELDUNG

(21) Anmeldenummer: 85101597.4

(22) Anmeldetag: 14.02.85

(51) Int. Cl.⁴: **B 01 D 13/00**

(30) Priorität: 08.03.84 DE 3408511
21.05.84 DE 3418870

(43) Veröffentlichungstag der Anmeldung:
25.09.85 Patentblatt 85/39

(84) Benannte Vertragsstaaten:
AT BE CH DE FR GB IT LI LU NL SE

(71) Anmelder: Akzo GmbH
Postfach 10 01 49 Kasinostrasse 19-23
D-5600 Wuppertal-1(DE)

(72) Erfinder: Klein, Wolfgang, Dr. Dipl.-Phys.
Am Hang 22
D-8753 Klingenberg(DE)

(72) Erfinder: Schneider, Klaus, Dr. Dipl.-Ing.
Am Stadtwald 62
D-8765 Erlenbach(DE)

(54) Verfahren zur Aufrechterhaltung einer Beladung mikroporöser Formkörper und Vorrichtung zur Durchführung des Verfahrens.

(57) Es wird ein Verfahren zur Aufrechterhaltung einer Beladung mikroporöser Formkörper für die Durchführung von Stoffaustauschprozessen beschrieben. Das Verfahren besteht darin, daß man während der Durchführung der Stoffaustauschprozesse dem Formkörper ein Beladungsmedium zuführt bzw. anbietet. Diese Zuführung erfolgt an einem nicht am Stoffaustauschprozeß beteiligten Teil der Formkörperoberfläche. Die Formkörper sind bevorzugt Membranen. Durch das Verfahren wird ein Verlust an dem in den Formkörperporen vorhandenen Medium ausgeglichen, ohne daß es eine Unterbrechung des Stoffaustauschprozesses bedarf.

Eine für die Durchführung des Verfahrens geeignete Vorrichtung wird ebenfalls beschrieben.

Das Verfahren läßt sich u.a. für controlled-release-, slow-release Prozesse sowie für Prozesse des erleichterten oder gekoppelten Transports von Metallionen vorteilhaft einsetzen.

EP 0 155 501 A2

A3GW32O83

## Verfahren zur Aufrechterhaltung einer Beladung mikroporöser Formkörper und Vorrichtung zur Durchführung des Verfahrens

A k z o   GmbH

Wuppertal

-.-.-.-.-

Die Erfindung betrifft ein Verfahren zur Aufrechterhaltung einer Beladung mikroporöser Formkörper für die Durchführung von Stoffaustauschprozessen.

Stoffaustauschprozesse, bei denen poröse Formkörper verwendet werden, sind bekannt und finden vielfach Anwendung im medizinischen, technischen und landwirtschaftlichen Bereich. Ein Beispiel hierfür sind slow-release bzw. controlled-release Prozesse, bei denen ein Wirkstoff, der in den Poren einer Trägersubstanz enthalten ist, langsam an die Umgebung abgegeben werden soll. Ein anderes Beispiel sind Transportverfahren wie der erleichterte oder gekoppelte Transport
von Metallionen durch die Wände poröser Membranen hindurch,
wie u.a. in der DE-A- 29 10 793 beschrieben. In diesem und
ähnlichen Verfahren befindet sich in den Membranporen ein
Komplexbildner, so daß der Transport der Metallionen über
ihre Komplexe erfolgt.

Ein Nachteil der erwähnten Verfahren besteht darin, daß
Verluste an demjenigen Medium auftreten, mit welchem die
Poren des Formkörpers bzw. der Membranen beladen sind.
Bei controlled-release Prozessen ist dieser Verlust durch
die Abgabe der Wirksubstanz an die Umgebung bedingt und
führt dazu, daß in gewissen Zeitabständen die Formkörper
regeneriert werden müssen. Daneben kann sich nach einiger
Zeit die Abgaberate in unerwünschter Weise verringern,
nämlich z.B. dann, wenn bereits ein erheblicher Teil der
Wirksubstanz abgegeben worden ist. Bei Verfahren des Transports von Substanzen, wie Metallionen, durch die Poren
einer Membran, wobei der Transport durch das in den Poren
vorhandene Medium vermittelt oder erleichtert wird, besteht
die Gefahr, daß nach längerer Laufzeit ein erheblicher Anteil dieser Substanz verloren geht. Dieser Effekt führt
anfangs zu einer Flußabnahme bei dem Prozeß, also einer
Verringerung der Menge in der Zeiteinheit transportierter
Metallionen. Schließlich muß das Verfahren abgebrochen werden
oder kommt von selbst zum Stillstand.

Diese bekannten Verfahren besitzen den Nachteil, daß die
Beladung der porösen Formkörper nicht während des Stoffaustausches aufrechterhalten werden kann. Vielmehr ist eine erneute Beladung des porösen Formkörpers nur nach dem Abbruch
bzw. bei einer Unterbrechung des Stoffaustauschprozesses
möglich. Ein neuerliches Beladen ist daher nicht nur vielfach
aufwendig, sondern verteuert in jedem Fall den Stoffaustauschprozeß.

Es stellte sich bei der vorliegenden Erfindung somit die
Aufgabe, ein Verfahren zu schaffen, bei dem eine Beladung
von mikroporösen Formkörpern während der Durchführung von
Stoffaustauschprozessen aufrechterhalten werden kann, ohne
den Stoffaustauschprozeß unterbrechen zu müssen, um somit
eine aufwendige Reinigung und diskontinuierliche erneute
Beladung des Formkörpers zu vermeiden und über die Aufrechterhaltung eines Beladungszustandes die Stoffaustauschprozesse
über sehr lange Laufzeiten ohne Abnahme der Effektivität
durchführen zu können. Ein weiterer Teil der Aufgabe bestand
darin, eine Vorrichtung zu schaffen, die die Durchführung
des Verfahrens ermöglicht.

Erfindungsgemäß besteht die Lösung der Aufgabe darin, daß
man als Formkörpermaterial ein Material mit einem durchgehenden Porensystem wählt und zur Aufrechterhaltung der
Beladung des Formkörpers während der Durchführung von
Stoffaustauschprozessen an der nicht am Stoffaustausch beteiligten Oberfläche des Formkörpers ein Beladungsmedium
zuführt.

Hierdurch wird, wie unten erläutert, gewährleistet, daß
während des Stoffaustauschprozesses das Beladungsmedium
oder Teile davon in das Porensystem des Formkörpers eindringen können, wenn Teile des Mediums, mit dem der Form-

0155501

körper bereits beladen ist, aus den Oberflächen des Formkörpers austreten.

Es wird somit die folgende Lehre gegeben:

Verfahren zur Aufrechterhaltung einer Beladung von
mikroporösen Formkörpern für die Durchführung von
Stoffaustauschprozessen, dadurch gekennzeichnet,
daß als Formkörpermaterial ein Material mit einem
durchgehenden Porensystem gewählt wird und daß zur
Aufrechterhaltung der Beladung während der Durchführung von Stoffaustauschprozessen an der nicht am
Stoffaustausch beteiligten Oberfläche der Formkörper
ein Beladungsmedium zugeführt wird.

Die Erfindung wird nunmehr im einzelnen beschrieben.

An einem Teil der Oberfläche eines mikroporösen Formkörpers, der nicht am Stoffaustausch beteiligt ist,
wird ein Beladungsmedium zugeführt. Es ist also für das
erfindungsgemäße Verfahren erforderlich, nicht die
gesamte Oberfläche des mikroporösen Formkörpers für
den Stoffaustausch zu verwenden, sondern einen Teil
der Oberfläche nicht daran zu beteiligen. Dieser Teil
der Oberfläche muß nicht groß sein. Es genügt in der Regel ein weit unter 50% der Gesamtoberfläche liegender Teil. Auf diese Weise kann der größte Teil der Oberfläche des Formkörpers für den Stoffaustausch benützt werden.

Das erfindungsgemäße Verfahren kann während der Durchführung von Stoffaustauschprozessen angewandt werden, bei denen mikroporöse Formkörper verwendet werden, in deren Poren sich ein Medium befindet. Beispiele für solche Stoffaustauschprozesse sind Transport- oder Stoffabgabeprozesse. Bei Transportprozessen findet z.B. die Abtrennung eines oder mehrerer Bestandteile aus einem System statt, das sich an einer der Formkörperoberflächen befindet. In diesem Fall sind die Formkörper in der Regel Membranen. Die selektiv abzutrennenden Bestandteile werden durch die Membran hindurch an die andere Oberfläche transportiert, wobei das in den Poren vorhandene Medium einerseits die Selektivität gewährleisten und andererseits den Transport erleichtern kann, z.B. über Komplexierung von Metallionen. Ein Beispiel für Abgabeprozesse sind controlled- bzw. slow-release-Verfahren, bei denen das in den Poren befindliche Medium an die Umgebung abgegeben wird.

Um diese Stoffaustauschprozesse über lange Zeit durchführen zu können, sieht das erfindungsgemäße Verfahren eine
Aufrechterhaltung einer Beladung der Formkörper vor. Darunter
ist zu verstehen, daß durch Zuführen eines Beladungsmediums
die Poren des Formkörpers mindestens teilweise gefüllt bzw.
beladen gehalten werden. Im Einzelfall muß demnach nicht
unbedingt das gesamte Porenvolumen von einem Medium gefüllt
gehalten werden, sondern nur mindestens zu einem solchen Anteil, welcher gewährleistet, daß der Stoffaustauschprozeß
ohne Einbuße an Effektivität über lange Zeit durchgeführt
werden kann. Insbesondere gewährleistet die Zuführung eines
Beladungsmediums während des Stoffaustauschprozesses, daß
eventuell auftretende Verluste an dem Medium, mit welchem
die Poren beladen, also ganz oder teilweise gefüllt sind,
durch das Beladungsmedium ausgeglichen werden können.

Unter Zuführen eines Beladungsmediums ist im Sinne der
Erfindung zu verstehen, daß während des Stoffaustausches
das Beladungsmedium dem Formkörper an einem nicht am Stoffaustausch beteiligten Teil seiner Oberfläche angeboten wird.
Es muß also im Einzelfall nicht ein vollkontinuierliches
Aufbringen des Beladungsmediums auf die Oberfläche stattfinden. Vielmehr kann ein diskontinuierliches Auftropfen
eines flüssigen Beladungsmediums oder ein diskontinuierliches
Zuführen eines Gases ausreichen.

Eine bevorzugte Ausführungsform des Verfahrens ist jedoch
dadurch gekennzeichnet, daß das Beladungsmedium kontinuierlich zugeführt wird. Eine weitere bevorzugte Ausführungsform besteht darin, an der für die Zuführung des Beladungsmediums vorgesehenen Oberfläche des Formkörpers ständig eine Schicht des Beladungsmediums aufrechtzuerhalten, z. B. dadurch, daß man an dieser Oberfläche einen ständigen Kontakt mit einem Reservoir an gasförmigem oder flüssigem Beladungsmedium aufrechterhält.

Durch das Anbieten von Beladungsmedium wird erreicht, daß dieses Medium in das Porensystem des Formkörpers eindringen kann, wenn der Beladungszustand des Formkörpers dies erfordert, wenn also Teile des Mediums, mit dem die Poren beladen sind, aus der Formkörperoberfläche austreten.

Ist das Beladungsmedium gasförmig, so kann dieses Eindringen in das Porensystem dadurch unterstützt werden, daß man einen Druck auf das Gas an der für die Zuführung vorgesehenen Oberfläche ausübt. Aus Kostengründen kann es hierbei von Vorteil sein, wenn man den statischen Druck des Gases selbst verwendet, z.B. durch Anbringen eines Reservoirs des Gases an einer Stelle, die höher liegt als die erwähnte Oberfläche, wobei das Reservoir Gas an diese Oberfläche nachliefern kann. Eine bevorzugte Ausführungsform des Verfahrens besteht also darin, auf die Schicht des Beladungsmediums einen Druck auszuüben, und in einer weiteren bevorzugten Ausführungsform ist dieser Druck ein statischer, der durch das Beladungsmedium selbst hervorgerufen wird. Dies gilt auch für den Fall, daß das Beladungsmedium eine Flüssigkeit ist.

In einer bevorzugten Ausführungsform besteht das Beladungsmedium aus den gleichen Komponenten wie das Medium, mit dem
das Porensystem bereits beladen ist, weist also die gleiche
qualitative Zusammensetzung auf. Im Einzelfall können jedoch
die qualitativen Zusammensetzungen voneinander abweichen,
z.B. dann, wenn durch das Beladungsmedium eine noch nicht
im Porensystem vorhandene Komponente zugeführt werden soll,
um Verfahrensbedingungen des Stoffaustauschprozesses bewußt
zu verändern.

In einer bevorzugten Ausführungsform ist das Beladungsmedium
eine Flüssigkeit, ebenso wie das bereits in den Poren vorhandene Medium. Neben der erwähnten Methode des Auftropfens
von Flüssigkeit auf einen Teil der Formkörperoberfläche
kommt in diesem Fall vor allem folgende Arbeitsweise für das
erfindungsgemäße Verfahren in Betracht: An einem Teil der
Oberfläche des Formkörpers, der nicht am Stoffaustauschprozeß beteiligt ist, befindet sich eine Schicht des
flüssigen Beladungsmediums, die wiederum mit einer größeren
Menge dieser Flüssigkeit in Verbindung steht, z.B. mit einem
Reservoir an dieser Flüssigkeit. Wenn der Beladungszustand
des Porensystems dies erfordert, kann Flüssigkeit aus der
erwähnten Schicht an der Oberfläche in das Porensystem eindringen, und durch das Reservoir wird Flüssigkeit an die
Oberflächenschicht nachgeliefert. Das Eindringen des
flüssigen Beladungsmediums in das Porensystem des Formkörpers kann auf mehrere Arten ermöglicht bzw. unterstützt
werden:

0155501

a) im einfachsten Fall ist der poröse Formkörper senkrecht
   angeordnet, un die Kontaktfläche zwischen Formkörper
   und Reservoirflüssigkeit befindet sich an seinem oberen
   Ende. In diesem Fall bewirkt oder unterstützt der hydrostatische Druck das Nachströmen von Flüssigkeit

b) in dem Reservoir und/oder an der Flüssigkeitsschicht
   an der vorgesehenen Formkörperoberfläche kann ein Überdruck an die Flüssigkeit angelegt werden

c) verwendet man für das Beladungsmedium eine Flüssigkeit,
   welche das Material des Formkörpers gut benetzt, so findet
   über Kapillarkräfte ein "Ansaugen" von Flüssigkeit statt,
   sobald Teile des in den Poren vorhandenen Mediums aus
   dem Porensystem austreten. Wenn im Beladungsmedium eine
   Komponente enthalten sein muß, die das Formkörpermaterial
   weniger gut benetzt, läßt sich gute Benetzung dadurch
   herstellen, daß man ein gut benetzendes Lösungsmittel
   zusetzt

d) das Nachströmen von Flüssigkeit aus der Oberflächenschicht
   in das Porensystem kann dadurch bewirkt oder unterstützt
   werden, daß man zusätzlich an einer nicht am Stoffaustausch beteiligten Oberfläche des Formkörpers einen
   Unterdruck an das Porensystem anlegt. Letzteres kann
   kontinuierlich oder diskontinuierlich erfolgen. Durch das
   Anlegen des Unterdrucks werden geringe Menge von Flüssigkeit aus den Poren abgezogen. Diese können jedoch aufgefangen und in das Rservoir an Beladungsmedium zurückgeführt werden. Die Unterstützung des Nachströmens durch
   Anlegen eines Unterdrucks führt zu einem schnelleren
   Nachströmen und damit zu einem schnelleren Ersatz eventuell

auftretender Verluste an Flüssigkeit in den Poren.

Eine bevorzugte Ausführungsform des Verfahrens ist also dadurch gekennzeichnet, daß an einer nicht am Stoffaustausch beteiligten Oberfläche des Formkörpers ein Unterdruck an das Porensystem angelegt wird. Das Abziehen von in den Poren enthaltenem Medium, beispielsweise durch Anlegen eines Unterdrucks, bietet andererseits neben schnellerem Eindringen des Beladungsmediums in das Porensystem den Vorteil, daß ein Medium in den Poren durch ein anderes ersetzt werden kann, wenn dies erwünscht ist, z.B. dann, wenn die Bedingungen des Stoffaustauschs geändert werden sollen. In einer bevorzugten Ausführungsform des Verfahrens wird also auch in dem mikroporösen Formkörper enthaltenes Medium während des Stoffaustauschprozesses abgezogen und ggf. dem Beladungsmedium zugeführt.

Der Teil der Formkörperoberfläche, wo das Beladungsmedium zugeführt wird, kann sich an einer beliebigen Stelle des Formkörpers befinden. Vorzugsweise liegt er jedoch an einem Ende des Formkörpers, so daß ein Nachströmen des Beladungsmediums nur in einer Richtung erfolgt.

Besonders bei großer Ausdehnung der Formkörper kann es von Vorteil sein, an dessen beiden Enden an je einem Teil der Oberfläche Beladungsmedium zuzuführen, so daß ein schnellerer Ausgleich eventuell auftretender Verluste des Mediums in den Poren oder Teilen davon erfolgen kann. In diesem Fall können an beiden erwähnten Teilen der Oberfläche Flüssigkeitsschichten vorliegen, die von einem gemeinsamen Reservoir an Beladungsmedium gespeist werden, und es kann ebenfalls

ein gewisser Überdruck dazu verwendet werden, das Nachströmen schneller zu bewerkstelligen. Gegebenenfalls befindet sich hierbei etwa in der Mitte zwischen den beiden
erwähnten Teilen der Oberfläche eine Zone an der Oberfläche
des Formkörpers, die von der Stoffaustauschoberfläche durch
Trennelemente abgetrennt ist. An dieser Zone kann dann
eine Auffangvorrichtung für Flüssigkeit angeschlossen sein,
um infolge Überdrucks eventuell aus dem Porensystem austretende geringe Mengen an Flüssigkeit zurückzugewinnen und
in das Reservoir zurückzuführen.

Der poröse Formkörper kann, wie erwähnt, senkrecht angeordnet sein, um durch hydrostatischen Druck das Nachströmen
von Beladungsmedium zu bewirken oder zu unterstützen. Bei
Durchführung von controlled-release Prozessen kann jedoch
eine horizontale Anordnung im Einzelfall günstiger sein,
um mit dem Beladungsmedium keinen Druck auf das in den
Poren befindliche Medium auszuüben und so eine sehr langsame Abgaberate dieses Mediums oder Teilen davon an die
Umgebung einzustellen. In diesem Fall kann das Nachströmen
von Flüssigkeit durch Kapillarwirkung allein erfolgen.
Voraussetzung ist hierfür eine gute Benetzung des Beladungsmediums gegenüber dem Formkörpermaterial.

Bei dem erfindungsgemäßen Verfahren werden mikroporöse
Formkörper verwendet. Unter mikroporösen Formkörpern im
Sinne der Erfindung werden hierbei Formkörper verstanden,
welche keine Poren aufweisen, deren mittlerer Durchmesser größer
als etwa 10 µm ist. Mikroporös im Sinne der Erfindung sind
auch Formkörper, welche keine Poren im eigentlichen Sinn mehr aufweisen, z.B. Formkörper, welche eine Struktur aufweisen, wie sie Ultrafiltrations- bzw. Dialysemembranen
besitzen.. Solche Formkörper, welche keine Öffnungen oder
Poren besitzen, deren Durchmesser größer ist als etwa
800 - 1 000 $\overset{o}{A}$, sind prinzipiell auch für das erfindungsgemäße Verfahren geeignet, da sie für Stoffaustauschprozesse
verwendet werden. Im Einzelfall muß die Auswahl des mikroporösen Formkörpers für das erfindungsgemäße Verfahren auf
die Art des Beladungsmediums abgestimmt sein, da dieses
in die Struktur des Formkörpers eindringen muß. Ist das
Beladungsmedium gasförmig, kommen vor allem mikroporöse
Formkörper in Betracht, welche keine Öffnungen oder Poren
besitzen, deren Durchmesser größer ist als etwa 1 000 $\overset{o}{A}$.
Ist das Beladungsmedium flüssig, wie es eine bevorzugte
Ausführungsform vorsieht, ist es in der Regel erforderlich,
daß der Formkörper definierte Poren aufweist. Deren mittlere
Durchmesser liegen in diesem Fall etwa zwischen 0,05 µm
und 10 µm. Hierdurch wird gewährleistet, daß das flüssige
Beladungsmedium in das Porensystem eindringen kann.

Eine bevorzugte Ausführungsform des Verfahrens besteht
darin, daß man Formkörper mit einer Porenstruktur verwendet, bei der neben Poren auch Zellen vorhanden sind.
Solche Formkörper lassen sich gut für Stoffaustauschprozesse
verwenden. Die annähernd kugelförmigen Zellen besitzen dabei
in der Regel mittlere Durchmesser von etwa 0,5 µm bis etwa
100 µm, vorzugsweise 1 bis 20 µm. Auf diese Weise kann
die Menge des im Formkörper gespeicherten Mediums erhöht
werden gegenüber Formkörpern, welche ein geringeres Porenvolumen
aufweisen. Eine Erhöhung der Menge an Medium im Porensystem ist erstrebenswert. So wird nämlich der Gefahr begegnet, daß Verluste an diesem Medium schneller eintreten,
als durch das Beladungsmedium, z.B. durch Kapillarkräfte,
nachgeliefert werden kann. Zwischen den Zellen sind Verbindungskanäle, die Poren, vorhanden. Diese stellen die
engsten Stellen für den Stofftransport dar und gewährleisten
z.B., daß bei Stoffaustauschprozessen keine Verunreinigungen
in Form größerer Teilchen von einer Oberfläche des Formkörpers durch seine Wand hindurch an die andere Oberfläche
gelangen können. Diese Poren besitzen mittlere Durchmesser,
welche um den Faktor 2 bis 200, vorzugsweise um den Faktor
5 bis 40 kleiner sind als die Durchmesser der kugelförmigen
Zellen. Die mittleren Porendurchmesser liegen vorzugsweise
bei etwa 0,05 bis etwa 10 µm.

Die verwendeten Formkörper müssen, um das Eindringen von
Behandlungsmedium von der Oberfläche des Formkörpers
in das gesamte Porensystem zu ermöglichen, ein durchgehendes
Porensystem besitzen. Das bedeutet, daß über die Poren bzw.
Verbindungskanäle ein Nachströmen von Behandlungsmedium
praktisch in das gesamte Porensystem möglich sein muß.
Im Einzelfall kann es vorkommen, wie z.B. bei controlled-
release Prozessen, daß einige wenige Poren, die keine Verbindung zum Gesamtporensystem besitzen, sich nicht störend
auswirken. Diese stehen zwar für das erfindungsgemäße Verfahren dann nicht zur Verfügung; da ihr Beitrag zur Wirkstoffabgabe aber wegen ihrer geringen Zahl unerheblich ist,
fällt es nicht ins Gewicht, wenn die Wirksubstanz in ihnen
nicht ersetzt werden kann. Der überwiegende Teil, vorzugsweise die Gesamtheit aller Poren muß jedoch in Verbindung
zueinander und zum Behandlungsmedium an der für die Zuführung vorgesehenen Oberfläche des Formkörpers stehen.

Die mikroporösen Formkörper sind in einer bevorzugten
Ausführungsform des Verfahrens Membranen in Form von Flachfolien, Schläuchen, Rohren oder Hohlfäden, wie sie für
Stoffaustauschprozesse und Trennverfahren eingesetzt werden.

Geeignete mikroporöse Membranen mit einer durchgehenden
Porenstruktur, wobei das Porensystem Zellen und Verbindungskanäle, die eigentlichen Poren, aufweist, sind
u.a. in der DE-OS 27 37 745 beschrieben; die entsprechenden Ausführungen dieser Schrift werden zum Inhalt der
hier vorliegenden Anmeldung gemacht. Bevorzugt weisen die
verwendeten Membranen ein hohes Porenvolumen auf, d.h.
einen hohen Anteil des durch die Poren und Zellen zusammen
gebildeten Volumen, bezogen auf das Gesamtvolumen der Membran.
Hohe Porenvolumina von 70 - 80% sind für Stoffaustauschprozesse besonders geeignet, da sie wegen der größeren
Menge des in den Poren vorhandenen Mediums zu einer Erhöhung der Effektivität führen. Bei Verfahren des erleichterten oder gekoppelten Transports von Metallionen
z.B. kann dann eine größere Menge an Komplexbildner in den
Poren untergebracht werden, wodurch die Menge der in der
Zeiteinheit über Komplexbildung transportierten Metallionen
ansteigt. Eine bevorzugte Ausführungsform des Verfahrens
sieht daher vor, daß die verwendeten Formkörper ein Poren -
volumen von 70 - 80% aufweisen.

Die Wandstärke der mikroporösen Membranen, die für das
erfindungsgemäße Verfahren besonders gut geeignet sind,
kann in weiten Bereichen variieren. In einer bevorzugten
Ausführungsform liegt sie im Bereich zwischen 100 und 300 µm.

Bei dickeren Wandstärken läßt sich zwar das erfindungsgemäße Verfahren ebenso durchführen; jedoch führen dicke
Wandstärken z.B. bei Verfahren des erleichterten oder gekoppelten Transports von Metallionen zu einer Verlängerung
der Strecke, welche die komplexierten Metallionen in der
Membranwand durch Diffusion zurückzulegen haben. Dies
resultiert in einer unerwünschten Verlangsamung des
Prozesses. Bei Wandstärken von weniger als 100 µm können
unter Umständen Probleme bezüglich mechanischer Stabilität vor allem beim Anlegen eines Über- oder Unterdrucks
entstehen. Dies gilt in erster Linie dann, wenn die verwendeten Membranen ein großes Porenvolumen, das oft bei
70 - 80% des Gesamtvolumens liegt, aufweisen.

Wenn das erfindungsgemäße Verfahren für controlled-release
oder slow-release Prozesse verwendet werden soll, brauchen
die mikroporösen Formkörper keine Membranen zu sein. Soll
z.B. ein Duftstoff über längere Zeit langsam an die umgebende Atmosphäre abgegeben werden, kann man einen mikroporösen stabförmigen Formkörper größerer Dicke verwenden,
an dessen einem Ende an einem Teil der Oberfläche Beladungsmedium zugeführt wird, das keinen Kontakt zur Umgebung
besitzt. Im Beladungsmedium muß dann für Druckausgleich
gesorgt werden, z.B. indem man einen kleinen Teil der
Oberfläche eines Reservoirs, in dem Beladungsmedium enthalten ist, in Form einer für Luft, nicht aber für das
Beladungsmedium permeablen Membran ausführt.

In vielen Fällen ist es günstig, in manchen sogar unentbehrlich, daß die mikroporösen Formkörper bzw. Membranen
innere Oberflächen aus hydrophobem Material besitzen.

Dies ist z.B. der Fall bei Verfahren des erleichterten
oder gekoppelten Transports von Metallionen, der über
Komplexierung erfolgt, wobei ein Komplexbildner sich in
den Poren befindet. Es gilt hier zu verhindern, daß die
wäßrigen Lösungen, welche Metallionen aufnehmen bzw.
abgeben und die sich an den Membranoberflächen befinden,
in das Porensystem eindringen und den Komplexbildner
daraus verdrängen. Diese Forderung läßt sich z.B. erfüllen durch Verwendung eines einheitlichen hydrophoben
Materials, in dessen Poren Wasser erst bei deutlich
höheren Drucken eindringen kann als eine unpolare organische Flüssigkeit. Eine andere Möglichkeit besteht darin,
daß nur die inneren Oberflächen des Formkörpers hydrophob sind, das sind die Oberflächen, welche die Poren
und, falls vorhanden, auch die Zellen besitzen. Falls
Formkörper, deren Material nicht hydrophob ist, verwendet
werden sollen, kann die innere Oberfläche hydrophobiert
werden, z.B. durch eine hydrophobe Beschichtung, die
durch Imprägnieren aufgebracht wird. Die Verwendung eines
Formkörpers mit hydrophober innerer Oberfläche beim erfindungsgemäßen Verfahren stellt eine bevorzugte Ausfführungsform dar und führt zu einer guten Benetzung durch
ein hydrophobes, unpolares, flüssiges Beladungsmedium.
Diese Flüssigkeit dringt daher leicht in das Porensystem
ein, und das Nachströmen von der Oberfläche des Formkörpers
in das Porensystem erfolgt, wie oben erwähnt, normalerweise bereits durch Kapillarkräfte, ohne daß es eines
Überdruckes bedarf. In einer bevorzugten Ausführungsform
des erfindungsgemäßen Verfahrens wird somit als Beladungsmedium eine Flüssigkeit verwendet, welche das Formkörpermaterial gut benetzt. Gute Benetzung liegt dann vor, wenn
ein Tropfen des flüssigen Beladungsmediums, auf eine
ebene Oberfläche des Formkörpers aufgebracht, gut
spreitet, d.h. einen Randwinkel von weniger als etwa 20°
aufweist. Bei idealer vollständiger Benetzung beträgt
dieser Winkel 0°.

In einer bevorzugten Ausführungsform besteht der Formkörper aus Polypropylen.

Als besonders geeignet für das erfindungsgemäße Verfahren
haben sich Formkörper bzw. Membranen aus mikroporösem
Polypropylen erwiesen, z.B. aus Accurel ® (Fa. Enka AG,
Wuppertal). Andere geeignete Materialien sind z.B. andere
Polyolefine und halogenierte Polyolefine.

Wie bereits erwähnt, besitzt das Beladungsmedium vorzugsweise qualitativ die gleiche Zusammensetzung  wie das
Medium, mit dem die Poren bereits beladen sind. Sind beide
ein Gemisch aus zwei oder mehr Komponenten, so ist es
jedoch oft von Vorteil, wenn das Beladungsmedium eine
andere quantitative Zusammensetzung aufweist als das Medium
in den Poren. Es kann nämlich der Fall eintreten, daß
eine Komponente des in den Poren vorhandenen Mediums etwas
leichter aus dem Porensystem austritt als andere. Dann
muß vor allem diese Komponente durch das Beladungsmedium
nachgeliefert werden. Ein Beispiel für diesen Fall ist
wieder der erwähnte erleichterte oder gekoppelte Transport
von Metallionen. Hier besteht das Medium oft aus einem
unpolaren Lösungsmittel und einem darin gelösten Komplexbildner, der komplexbildende, also in einem gewissen Ausmaß polare Eigenschaften aufweist. Die hierdurch hervorgerufene
geringe Löslichkeit des Komplexbildners in den wäßrigen
Lösungen an den Membranoberflächen kann dazu führen, daß
zwar das unpolare Lösungsmittel in den Poren erhalten
bleibt, die Menge an Komplexbildner in den Poren aber langsam abnimmt. In diesem Fall muß Komplexbildner, vor allem
auch durch Diffusion, aus dem Beladungsmedium nachgeliefert werden. Um die Geschwindigkeit dieser Diffusion
zu erhöhen, empfiehlt es sich, die Konzentration an
Komplexbildner im Beladungsmedium höher einzustellen als
in dem Medium, welches in den Poren vorliegt.

Eine bevorzugte Ausführungsform des Verfahrens ist somit
dadurch gekennzeichnet, daß das Beladungsmedium ein Gemisch
aus zwei oder mehr Komponenten ist, wobei mindestens eine
Komponente im Beladungsmedium in höherer Konzentration
vorliegt als in dem Medium, mit dem die Poren beladen sind.
Daneben ist es in dem Fall, wo speziell eine Komponente
z.B. durch Diffusion nachzuliefern ist, auch zweckmäßig,
diskontinuierlich oder kontinuierlich einen leichten Unterdruck an das Porensystem anzulegen. Hierzu ist es von
Vorteil, wenn sich die Kontaktfläche zwischen Beladungsmedium und Formkörper an einem Ende des Formkörpers und
die Stelle, wo Unterdruck angelegt wird, am entgegengesetzten Ende befindet. So wird ein schnelles Nachströmen
dieser Komponente ermöglicht.

Das erfindungsgemäße Verfahren kann bei allen Prozessen
verwendet werden, in denen mikroporöse Formkörper der
beschriebenen Art eingesetzt werden, und bei denen der Fall auftreten kann, daß Teile des in den Poren vorhandenen
Mediums aus den Poren austreten, deren Nachlieferung während
des Prozesses erwünscht ist. Die Formkörper müssen hierzu
von ihrer Ausführung bzw. Dimensionierung her so geschaffen
sein, daß an einem Teil ihrer Oberfläche, der nicht am
Stoffaustausch beteiligt ist, Beladungsmedium zugeführt
werden kann. Insbesondere besteht eine bevorzugte Ausführungsform des erfindungsgemäßen Verfahrens darin, es bei der
Durchführung von Transportprozessen einzusetzen. Ein Prozeß,
für den sich das erfindungsgemäße Verfahren gut eignet,
ist der erleichterte oder gekoppelte Transport von Metallionen. Hierbei werden mikroporöse Membranen mit hydrophober
innerer  Oberfläche verwendet, in deren Poren sich mindestens
ein Komplexbildner befindet, der höchstens geringe Löslichkeit in Wasser besitzt. An einer der Membranoberflächen
befindet sich die Metallionen abgebende, an der anderen die
Metallionen aufnehmende wäßrige Lösung. Die beiden wäßrigen
Lösungen weisen einen Druck auf, der niedriger ist als
derjenige (sog. liquid entry pressure), der mindestens erforderlich ist, damit sie in die Poren eindringen können.
Tritt, z.B. wegen einer gewissen Löslichkeit in Wasser,
während des Prozesses Komplexbildner aus den Poren aus,
wird er durch das Beladungsmedium ersetzt. So gelingt es,
eine wesentlich längere Laufzeit der Membran zu erreichen,
als bei bekannten Verfahren, die abgebrochen werden müssen,
sobald die Verluste an Komplexbildner einen gewissen Wert
überschreiten.

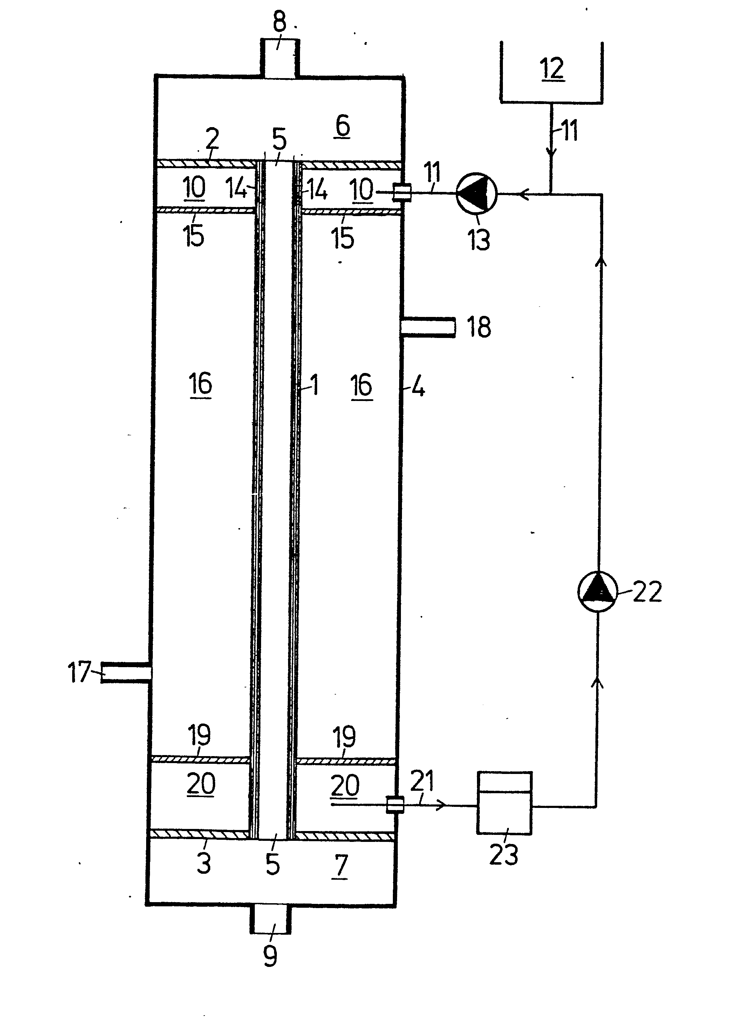

Eine besonders bevorzugte Vorrichtung zur Durchführung des
erfindungsgemäßen Verfahrens enthält ein Bündel von
parallel ausgerichteten mikroporösen Hohlfäden, die mittels einer Einbettmasse in ein Gehäuse eingebaut sind. Die
Vorrichtung ist in Abbildung 1 vereinfacht schematisch
im Längsschnitt dargestellt, wobei zur besseren Veranschaulichung nur ein Hohlfaden gezeichnet ist.

In Abbildung 1 ist ein Hohlfaden 1 dargestellt, der mittels
Einbettmasse 2 und 3 in ein Gehäuse 4 eingebaut ist. Das
Lumen des Hohlfadens 5 steht in Verbindung mit je einer
Kammer 6 bzw. 7 am oberen und unteren Ende des Hohlfadens.
Die Kammern 6 und 7 sind mit einer Zu- bzw. Ableitung 8
und 9 für Flüssigkeit versehen.
Anschließend an die Einbettmasse 2 folgt eine Kammer 10,
welche durch die Einbettmasse 2 von der Kammer 6 so getrennt ist, daß zwischen beiden kein Flüssigkeitsaustausch und ggf. kein Gasaustausch stattfinden kann.
In der Kammer 10 befindet sich Beladungsmedium. In die
Kammer 10 führt eine Leitung 11, welche mit einem größeren
Behälter 12 für Beladungsmedium in Verbindung steht.
Zwischen Behälter 12 und Kammer 10 ist eine Pumpe 13
eingebaut, mittels derer ein Überdruck im Beladungsmedium
in der Kammer 10 erzeugt werden kann. Die Elemente 11,
12 und 13 sind für die Durchführung des erfindungsgemäßen
Verfahrens nicht unbedingt erforderlich. Je nach durchzuführendem Prozeß und Dimensionen der Vorrichtung kann
das Vorhandensein einer mit Flüssigkeit gefüllten Kammer
10 bereits genügen. Fallen die Elemente 11, 12, 13 fort,
ist die Kammer 10 durch das Gehäuse 4 nach außen abgeschlossen, wobei lediglich für Druckausgleich in der
Kammer zu sorgen ist, um das Nachströmen von Flüssigkeit
in das Porensystem zu ermöglichen.

Das Beladungsmedium in der Kammer 10 steht mit einem Teil 14 der Hohlfadenoberfläche in Kontakt. Die Kammer 10 ist nach unten durch ein Trennelement 15 von der nächsten Kammer 16 flüssigkeitsdicht oder ggf. gasdicht abgeschlossen. Das Trennelement 15 kann aus der gleichen Einbettmasse, z.B. Polyurethan, wie die Einbettmassen 2 und 3 bestehen. Die Kammer 16 steht für Stoffaustausch zur Verfügung und enthält eine Zu- und eine Ableitung (17 und 18) für Flüssigkeit. Die Kammer 16 ist nach unten flüssigkeitsdicht und ggf. gasdicht durch ein Trennelement 19 von der anschließenden Kammer 20 abgeschlossen. Das Trennelement 19 kann wiederum aus der erwähnten Einbettmasse bestehen. Von der Kammer 20 führt eine Leitung 21 für Flüssigkeit aus dem Gehäuse heraus. Die Leitung 21 führt zu einer Pumpe 22 und von dort in die Leitung 11. Auf diese Weise ist es möglich, aus dem Behälter 12 Flüssigkeit mittels einer Pumpe 13 und/oder 22 in die Kammern 10 und 20 zu pumpen und dort einen Überdruck aufzubauen.

Es kann aber auch eine Auffangvorrichtung 23 für Flüssigkeit vorgesehen sein, um aus dem Porensystem in die Kammer 20 austretende Flüssigkeit aufzufangen. In diesem Fall kann man die Pumpe 22 dazu benutzen, kontinuierlich oder diskontinuierlich einen Unterdruck in der Kammer 20 zu erzeugen und so das Nachströmen von Flüssigkeit aus der Kammer 10 in das Porensystem zu beschleunigen. Die Kammer 20 ist nach unten flüssigkeitsdicht und ggf. gasdicht von der Kammer 7 durch die Einbettmasse 3 abgeschlossen. Die Kammer 7 steht mit dem Lumen 5 des Hohlfadens in Verbindung und enthält eine Flüssigkeitszu- bzw. -ableitung 9.

Gegebenenfalls enthält die Vorrichtung noch, nicht gezeichnete, Heizelemente, wie z.B. einen Heizmantel an
der äußeren Oberfläche des Gehäuses 4 oder Heizmäntel,
welche die Flüssigkeitsleitungen bzw. Gasleitungen umgeben.

Im Folgenden wird eine besonders geeignete Vorrichtung
zur Durchführung des erfindungsgemäßen Verfahrens beschrieben:

Vorrichtung zur Durchführung des Verfahrens enthaltend
ein Bündel von parallel ausgerichteten mikroporösen Hohlfäden 1 , welche mittels Einbettmasse 2 und 3 in ein
Gehäuse 4 eingebaut sind, wobei in Längsrichtung an der
äußeren Oberfläche der Hohlfäden nacheinander folgende
Elemente vorhanden sind:

a) eine Kammer 6 mit Zulaufeinheit 8 , welche mit den
   Lumina 5 der Hohlfäden in Verbindung steht

b) Einbettmasse 2

c) eine Kammer 10 , welche über eine Zuleitung 11
   mit einem Flüssigkeitsbehälter 12 in Verbindung
   stehen kann, wobei zwischen Kammer und Behälter eine
   Pumpe 13 angebracht sein kann

d) Trennelement 15

e) eine Kammer 16 , welche eine Zuleitung 17
   und eine Ableitung 18 aufweist

f) Trennelement 19

g) eine Kammer 20 , welche eine Ableitung 21
   aufweist, die mit der Kammer 10 in Verbindung
   stehen kann und wobei in der Ableitung eine Pumpe 22

eingebaut ist und wobei zwischen Kammer 20 und Pumpe 22 eine Auffangvorrichtung 23 für Flüssigkeit vorhanden sein kann

h) Einbettmasse 3

i) eine Kammer 7 mit Ablaufeinheit 9 , welche mit den Lumina 5 der Hohlfäden in Verbindung steht.

Führt man in der beschriebenen Vorrichtung einen erleichterten oder gekoppelten Transport von Metallionen durch, so tritt z.B. die Metallionen abgebende wäßrige Lösung oder Dispersion bei der Öffnung 17 in die Kammer 16 ein und gibt an der äußeren Hohlfadenoberfläche die Metallionen an das Medium in den Poren ab, das einen Komplexbildner enthält. Die Metallionen diffundieren durch die Hohlfadenwand in Form ihrer Komplexe und werden an der inneren Oberfläche an eine im Lumen 5 strömende wäßrige Flüssigkeit abgegeben. Die Zirkulierung der wäßrigen Flüssigkeiten ist hier von Vorteil. Die Metallionen abgebende Flüssigkeit verläßt die Kammer 16 bei der Öffnung 18, die Metallionen aufnehmende Flüssigkeit tritt bei der Öffnung 8 in die Kammer 6 ein, durchströmt das Lumen 5 und verläßt die Kammer 7 bei der Öffnung 9. Die Zu- bzw. Ableitung 17 bzw. 18 befinden sich bevorzugt an entgegengesetzten Enden der Kammer 16.

Verwendet man das erfindungsgemäße Verfahren zu slow- bzw. controlled-release Prozessen, bei denen Wirkstoffe, z.B. an die umgebende Luft, abgegeben werden, verwendet man zweckmäßigerweise eine einfachere Vorrichtung. Die mikroporösen Formkörper können dann z.B. als stabförmige Körper ausgebildet sein, deren Herstellung billiger ist als die von Hohlfäden. Das Gehäuse 4 sowie die Kammern 6 und 7 und gegebenenfalls die Kammer 20 können dann wegfallen, so daß sich an dem Formkörper nur die Kammer 10
befindet, die gegebenenfalls über eine Leitung 11 mit
einem größeren Behälter in Verbindung steht.

Die Erfindung wird nun durch ein Beispiel veranschaulicht.

Beispiel
─────────

Verwendet wurde ein Glasgefäß, das oben in einem Hals
endete. Durch den Hals wurde ein an beiden Enden offener
Hohlfaden senkrecht in das Glasgefäß eingeführt, so daß
das untere Ende des Hohlfadens den Gefäßboden nicht
berührte. Mittels einer Einbettmasse wurde der Hohlfaden
mit dem Hals des Gefäßes verbunden. Die Einbettmasse war
soweit unter dem oberen Rand des Halses angebracht, daß
ein Behälter gebildet wurde, der nach unten durch die
Einbettmasse, an der Innenseite durch den Hohlfaden und
an der Außenseite durch den Hals des Glasgefäßes abgeschlossen war. Nach oben war der Behälter offen. Dieser
Behälter diente als Flüssigkeitsreservoir, welches mit
dem Beladungsmedium gefüllt werden konnte. Der Hohlfaden
bestand aus mikroporösem Polypropylen mit einem inneren
Durchmesser von 1.8 mm und einem äußeren Durchmesser von
2.6 mm. Das Polypropylen wies eine durchgehende Porenstruktur mit Zellen und Verbindungsporen auf.

Der maximale Durchmesser der engsten Stellen der Verbindungskanäle (Poren) betrug ca. 0,55 µm, gemessen mit
der Blaspunktmethode.

Es wurden mehrere Versuche durchgeführt. Zu Beginn eines jeden Versuchs wurde das Porensystem des Hohlfadens durch Imprägnieren mit einer 9.5 (Gew.-%) prozentigen Lösung von 2-Hydroxy-5-nonyl-acetophenonoxim in Cyclohexan gefüllt. Das Glasgefäß war bis knapp unterhalb der Einbettmasse mit einer wäßrigen $CuSO_4$-Lösung gefüllt, so daß der größte Teil des unten aus der Einbettmasse herausragenden Hohlfadens an seiner äußeren Oberfläche und im Lumen in Kontakt mit der Salzlösung stand. Die Salzlösung wurde kontinuierlich durch das Lumen mit einer Geschwindigkeit von ca. 1 m/sec nach oben gepumpt und anschließend wieder in das Gefäß zurückgeführt.

In einem ersten Versuch, der als Vergleichsversuch diente, wurde mit leerem Reservoir, d.h. ohne Zuführen eines Beladungsmediums gearbeitet. In gewissen Zeitabständen wurden kleine Stücke am unteren Ende des Hohlfadens abgeschnitten und an diesen der Gehalt an dem substituierten Oxim nach Extraktion UV-spektroskopisch bestimmt. Es ergab sich, daß der Gehalt vom Anfangswert bis zu einer Laufzeit von etwa 700 Stunden annähernd linear auf etwa 50% des Anfangswerts abfiel

In weiteren Versuchen wurde das Reservoir mit einer 59 (Gew.-%) prozentigen Lösung des Oxims in Cyclohexan gefüllt. Es wurde wie im Vergleichsversuch verfahren, wobei die Flüssigkeit im Reservoir ausschließlich auf Grund von Kapillarkräften, Diffusion und hydrostatischem Druck in das Porensystem nachströmen konnte. Es wurde nach ca. 100 Stunden Laufzeit eine Zunahme des Gehalts an Oxim am unteren Ende des Hohlfadens um etwa 10% bestimmt. Nach 300 Stunden betrug die Zunahme etwa 20%, nach 500 Stunden etwa 50%. Von da an war kaum noch eine Zunahme zu verzeichnen, der Gehalt blieb in etwa konstant.

Diese Ergebnisse waren in mehreren Versuchen mit nur geringen Abweichungen reproduzierbar.

- 01 -        A3GW32083

Patentansprüche

1. Verfahren zur Aufrechterhaltung einer Beladung von
   mikroporösen Formkörpern für die Durchführung von
   Stoffaustauschprozessen, dadurch gekennzeichnet,
   daß als Formkörpermaterial ein Material mit einem
   durchgehenden Porensystem gewählt wird und daß zur
   Aufrechterhaltung der Beladung während der Durchführung von Stoffaustauschprozessen an der nicht am
   Stoffaustausch beteiligten Oberfläche der Formkörper
   ein Beladungsmedium zugeführt wird.

2. Verfahren nach Anspruch 1, dadurch gekennzeichnet,
   daß das Beladungsmedium aus den gleichen Komponenten
   besteht wie das Medium, mit dem das Porensystem bereits
   beladen ist.

3. Verfahren nach Anspruch 1 und 2, dadurch gekennzeichnet,
   daß das Beladungsmedium kontinuierlich zugeführt wird.

A3GW32083

4. Verfahren nach Anspruch 1 bis 3, dadurch gekennzeichnet, daß an der für die Zuführung des Beladungsmediums vorgesehenen Formkörperoberfläche eine Schicht dieses Mediums ständig aufrechterhalten wird.

5. Verfahren nach Anspruch 4, dadurch gekenneichnet, daß auf die Schicht des Beladungsmediums ein Druck ausgeübt wird.

6. Verfahren nach Anspruch 5, dadurch gekennzeichnet, daß der Druck ein statischer, durch das Beladungsmedium hervorgerufener Druck ist.

7. Verfahren nach Anspruch 1 bis 6, dadurch gekennzeichnet, daß an einer nicht am Stoffaustausch beteiligten Oberfläche des Formkörpers ein Unterdruck an das Porensystem ausgelegt wird.

8. Verfahren nach Anspruch 1 bis 7, dadurch gekennzeichnet, daß in dem mikroporösen Formkörper enthaltenes Medium während des Stoffaustauschprozesses auch abgezogen und gegebenenfalls dem Beladungsmedium zugeführt wird.

9. Verfahren nach Anspruch 1 bis 8, dadurch gekennzeichnet, daß das Beladungsmedium ein Gemisch aus zwei oder mehr Komponenten ist, wobei mindestens eine Komponente im Beladungsmedium in höherer Konzentration vorliegt, als in dem Medium, mit dem die Poren beladen sind.

10. Verfahren nach Anspruch 1 bis 9, dadurch gekennzeichnet, daß das Beladungsmedium éine Flüssigkeit ist.

A3GW320 83

11. Verfahren nach Anspruch 10, dadurch gekennzeichnet, daß eine Flüssigkeit verwendet wird, welche das Formkörpermaterial gut benetzt.

12. Verfahren nach Anspruch 1 bis 11, dadurch gekennzeichnet, daß der Formkörper eine hydrophobe innere Oberfläche aufweist.

13. Verfahren nach Anspruch 1 bis 12, dadurch gekennzeichnet, daß der Formkörper aus Polypropylen besteht.

14. Verfahren nach Anspruch 1 bis 13, dadurch gekennzeichnet, daß als Formkörper Membranen in Form von Flachfolien, Schläuchen, Rohren oder Hohlfäden verwendet werden.

15. Verfahren nach Anspruch 14, dadurch gekennzeichnet, daß die Membranen eine Wandstärke im Bereich von 100 µm bis 300 µm aufweisen.

16. Verfahren nach Anspruch 1 bis 15, dadurch gekennzeichnet, daß die Formkörper ein Porensystem aufweisen, das Zellen und Verbindungskanäle zwischen den Zellen enthält.

17. Verfahren nach Anspruch 1 bis 16, dadurch gekennzeichnet, daß die Formkörper ein Porenvolumen von 70 - 80% aufweisen.

18. Vorrichtung zur Durchführung des Verfahrens nach den Ansprüchen 1 bis 17 enthaltend ein Bündel von parallel ausgerichteten mikroporösen Hohlfäden (1), welche

0155501

A3GW32083

mittels Einbettmasse (2 und 3) in ein Gehäuse (4) eingebaut sind, wobei in Längsrichtung an der äußeren Oberfläche der Hohlfäden nacheinander folgende Elemente vorhanden sind:

a) eine Kammer (6) mit Zulaufeinheit (8), welche mit den Lumina (5) der Hohlfäden in Verbindung steht

b) Einbettmasse (2)

c) eine Kammer (10), welche über eine Zuleitung (11) mit einem Flüssigkeitsbehälter (12) in Verbindung stehen kann, wobei zwischen Kammer und Behälter eine Pumpe (13) angebracht sein kann

d) Trennelement (15)

e) eine Kammer (16), welche eine Zuleitung (17) und eine Ableitung (18) aufweist

f) Trennelement (19)

g) eine Kammer (20), welche eine Ableitung (21) aufweist, die mit der Kammer (10) in Verbindung stehen kann und wobei in der Ableitung eine Pumpe (22) eingebaut ist und wobei zwischen Kammer (20) und Pumpe (22) eine Auffangvorrichtung (23) für Flüssigkeit vorhanden sein kann

h) Einbettmasse (3)

i) eine Kammer (7) mit Ablaufeinheit (9), welche mit den Lumina (5) der Hohlfäden in Verbindung steht.

0155501

A3GW320 83

19. Verwendung des Verfahrens nach Anspruch 1 bis 17, bei der Durchführung von Transportprozessen.

20. Verwendung des Verfahrens nach Anspruch 19 bei der Durchführung von Prozessen des erleichterten oder gekoppelten Transports von Metallionen.